# EUROPEAN PATENT SPECIFICATION

(11) **EP 4 082 606 B1**
(45) Date of publication and mention of the grant of the patent: **26.03.2025**
(21) Application number: 21201182.9
(22) Date of filing: 06.10.2021
(51) Int. Cl.: A61N 5/06, F21S 8/06, F21V 33/00, F21Y 113/10

(54) **LED-MODULE AND LIGHTING SYSTEM WITH SUCH LED-MODULE EMITTING LIGHT HAVING A UV-B COMPONENT**
LED-MODUL UND BELEUCHTUNGSSYSTEM MIT EINEM SOLCHEN LED-MODUL, DAS LICHT MIT EINER UV-B-KOMPONENTE AUSSENDET
MODULE À DEL ET SYSTÈME D'ÉCLAIRAGE COMPORTANT UN TEL MODULE À DEL ÉMETTANT DE LA LUMIÈRE AYANT UN COMPOSANT UV-B

(30) Priority: 30.04.2021 EP 21171544
(43) Date of publication of application: 02.11.2022
(73) Proprietor: Tridonic GmbH & Co. KG, 6851 Dornbirn (AT)
(72) Inventor: Dobos, Janos, 6851 Dornbirn (AT); Meitz, Simon Michael, 6851 Dornbirn (AT)
(74) Representative: Beder, Jens

(56) References cited:
- WO-A1-2021/005029
- WO-A1-2021/148580
- KR-A- 20170 120 772
- KR-A- 20200 074 440
- US-A1- 2018 172 219

## Description

The invention regards an LED-module and a lighting system using such a LED-module, wherein the LED-module emits light having a UV-B component for enhancing human production of vitamin D in the skin.

For human health and well-being it is important that enough vitamin D is available. Vitamin D serves a plurality of purposes in the human body, for example, regulating the absorption of calcium and phosphorus and facilitating normal immune system function, which is extremely important in similar pandemic situations like today. The daily amount of vitamin D needed by a human is estimated to be around 20 µg. Vitamin D is included in the plurality of different food products and can also be supplemented by dietary supplements. Apart from that, the human body is able to produce vitamin D in response to exposing the skin to UV-B radiation. However, the spectrum in which the skin is responsive to UV-B radiation is rather narrow so that even natural sunlight does not provide sufficient UV-B radiation to cause the human body to generate vitamin D during wintertime.

Nowadays, the problem gets even worse because many people do not spend much time outside in natural sunlight. One reason for that is that people in modern societies rather spend more time indoors maybe even because of the risk of skin cancer, which is also caused by UV-B radiation included in sunlight. Compensating this reduction of a production of vitamin D by dietary supplements requires the people being aware of the fact that their need of vitamin D is higher than what they actually ingest.

Over the last years, light therapies using dedicated narrowband light sources for quite a number of different diseases became more and more popular. However, a lack of vitamin D is not really a disease in first place and applying a therapy does not seem suitable to help compensating for a general lack of exposure to sunlight. Such lack of exposure to sunlight is not only a problem for people who do spend most of the time indoors but may also be caused by winter season or longer foggy time periods when the access to natural light is very limited.

Thus, it would be desirable to increase the production of vitamin D by the human body without the need of applying a dedicated therapy or changing the habits, in particular the eating habits.

The present invention solves this problem by the LED-module and the lighting system according to the independent claims. The LED-module and the respective lighting system increase the exposure of the skin of people to light in a wavelength range suitable for the production of vitamin D by the human body. The invention proposes to provide an LED-module which, in addition to one or more first type LEDs, which emit radiation suitable for generating white light, comprises at least one second type LED emitting UV-B radiation that is dedicated for increasing vitamin D production. As a result, the entire LED-module emits white light for illuminating an area or room but additionally UV-B radiation. Thus, the LED-module, and the lighting system comprising such LED-module, is suitable to expose the skin of humans to radiation having a wavelength range that causes an increased vitamin D production. This has the advantage, that in an area where people spend quite a lot of time, and without any need of changing their habits, the production of vitamin D is improved. Thus, the exposure time of the skin of people to UV-B radiation is significantly increased compared to the average exposure times in sunlight only. Document WO 2021/005029 discloses an indoor illumination system comprising LEDs emitting both white light for illumination and UV-B light for stimulation of vitamin D production. The power of the UV-B illumination should not exceed a safety threshold RG2.

The inventive LED-module comprises at least one first type LED which is suitable to generate white light for illuminating an area or room. It is to be noted that not necessarily only a single LED for generating the white light, but any combination of LEDs for commonly known techniques for generating white light for illuminating a room is suitable. The LED-module may even comprise a plurality of LEDs for generating white light in combination. The LED-module may produce white light with or without a converter to convert the radiation emitted by the at least one first type LED.

According to the invention, it is important that at least one second type LED is included in the LED-module, which emits dedicated UV-B radiation, which does not contribute to the generation of white light for illumination reasons. Although there are LED-modules known in the art, for example as disclosed in DE 20 2015 105 686 U1, that combine a plurality of different LEDs, one of the LEDs emitting radiation in the UV range, but in the end these modules are all designed for emitting only white light. Even the LEDs emitting radiation in the UV range in first place contribute to the generation of white light so that no or negligible UV-B radiation is emitted. Consequently, exposing the skin to light emitted by such LED-modules cannot enhance vitamin D production in the human body.

The LED-module may be realized as a chip-on-board module (COM), or one comprising surface mounted (SMD) LEDs
The dependent claims define advantageous embodiments and aspects.

According to one advantageous embodiment, the LED-module comprises an optical diffuser arranged such that radiation emitted by this at least one second type LED passes through the optical diffuser. Generally, UV-B radiation is not only advantageous for the human body but may also cause severe problems. Thus, it is necessary that the dose with which the skin, or, maybe even more important, the retina of the eye are exposed is not critical. According to this advantageous embodiments, the diffuser is used to diffuse the UV-B radiation emitted by the at least one second type LED. The diffuser may be attached to the LED-module in such a way that it covers the LED of the second type so that any radiation which is emitted by the at least one second type LED necessarily passes the diffuser.

Optionally, the optical diffuser is arranged such that radiation emitted by the at least one first type LED and radiation emitted by the at least one second type LED passes through the optical diffuser. Thus, the optical diffuser allows the radiation emitted by the at least one first type LED and the radiation emitted by the at least one second type LED to pass or transmit through it, while optically diffusing or scattering these radiations. Therefore, the optical diffuser allows the white light generated based on the radiation emitted by the at least one first type LED to pass through. In other words, the optical diffuser may be implemented such that it does not absorb a significant amount of the radiation emitted by the at least one first type LED and the radiation emitted by the at least one second type LED. The diffuser may be attached to the LED-module in such a way that it covers the at least one LED of the first type and the at least one LED of the second type so that any radiation which is emitted by the at least one first type LED and the at least one second type LED necessarily passes the diffuser.

Optionally, only a part of the optical diffuser may allow the radiation emitted by the at least one first type LED and the UV-B radiation emitted by the at least one second type LED to pass through it. That is, only a part of the optical diffuser may be made of a material allowing the radiation emitted by the at least one first type LED and the UV-B radiation emitted by the at least one second type LED to pass through it. This improves costs for implementing the LED-module, because a material allowing the radiation emitted by the at least one first type LED and the UV-B radiation to pass through may be expensive. The optical diffuser may be arranged, optionally may be attached to the LED-module, such that the radiation emitted by the at least one second type LED passes through the aforementioned part of the optical diffuser.

Due to arranging the optical diffuser such that radiation emitted by the at least one first type LED and radiation emitted by the at least one second type LED is optically diffused or scattered by the optical diffuser, both radiations may be evenly distributed in the direction of radiation. This guarantees an evenly distribution of the UV-B radiation emitted by the at least one second type LED at a location in the direction of radiation (e.g. beneath the LED-module). Therefore, this prevents dangerous hotspots of too high UV-B radiation and at the same time allows an effective vitamin D generation on the skin (not covered with UV-B absorbing clothing) of a person that is illuminated by the LED-module. The optical diffuser may be an optical diffuser sheet or optical diffuser plate.

In addition or alternatively, at least a part of the optical diffuser for the radiation emitted by the at least one second type LED may be made of a material having a transmittance in a wavelength range from 250 nm to 800 nm, optionally from 300 nm to 600 nm. Optionally, the optical diffuser is made of the material having a transmittance in a wavelength range from 250 nm to 800 nm, optionally from 300 nm to 600 nm.

Optionally, the transmittance of the aforementioned material is in a wavelength range having a lower wavelength boundary of 250 nm or 300 nm and an upper wavelength boundary of 600 nm or 800 nm. In an embodiment, the transmittance of the aforementioned material is for wavelengths equal to or greater than 250 nm. In another embodiment, the transmittance of the aforementioned material is for wavelengths equal to or greater than 300 nm.

With regard to a material, the term transmittance of the material for a radiation may indicate that the material allows the radiation to pass through or transmit through the material. In case the radiation (e.g. visible light) may not only pass through the material but also a person may see through the material, the material may be referred to as a transparent material. In case a material allows the radiation (e.g. visible light) to pass through it but a person cannot see through the material, the material may be referred to as a translucent material. Normal window glass is an example of a transparent material or transparent glass. Frosted glass is an example of a translucent material or a translucent glass.

The transmittance or the degree of transmittance of a material for a radiation, that allows the radiation to pass through it, may be indicated by the internal transmittance of the material. The internal transmittance refers to transmittance measured by excluding reflection losses at the entrance and exit surfaces of the material for a given thickness. The greater the internal transmittance the greater the percentage of radiation that exits the material with regard to the radiation that entered the material and vice versa. Thus, the greater the internal transmittance of a material with regard to a radiation, the lower the amount of the radiation that is absorbed by the material and vice versa. For example, a material having an internal transmittance (in short transmittance) of 90% may mean that 90% of radiation that entered the material exits the material again after having passed through the material.

In other words, at least a part of the optical diffuser for the radiation emitted by the at least one second type LED or the optical diffuser may be made of a material that has the same optical properties for the radiation emitted by the at least one first type LED and the radiation emitted by the at least one second type LED. With regard thereto, having a same optical property may mean that the material fulfils a minimum criterion regarding the optical property. For example, in case the optical property is transmittance, having the same optical property for the radiation emitted by the at least one first type LED and the radiation emitted by the at least one second type LED may mean that the material has an internal transmittance above a threshold for the internal transmittance, e.g. equal to or greater than 90%, for the radiation emitted by the at least one first type LED and the UV-B radiation emitted by the at least one second type LED. For example, the material may have an internal transmittance of at least 90% for the UV-B radiation emitted by the at least one second type LED and an internal transmittance of at least 99% for the radiation emitted by the at least one first type LED.

Optionally, the material is PMMA (polymethyl methacrylate) or glass. The term "acrylic material" may be used as a synonym for PMMA. Thus, according to an embodiment, at least the part of the optical diffuser for the UV-B radiation emitted by the second type LED may be made of PMMA having a transmittance in a wavelength range from 250 nm to 800 nm, optionally from 300 nm to 600 nm. Optionally, the optical diffuser may be made of the aforementioned material. According to another embodiment, at least the part of the optical diffuser for the UV-B radiation emitted by the second type LED may be made of glass having a transmittance in a wavelength range from 250 nm to 800 nm, optionally from 300 nm to 600 nm. Optionally, the optical diffuser may be made of the aforementioned material. Such a glass may have an internal transmittance of at least 90% for the UV-B radiation emitted by the at least one second type LED and an internal transmittance of at least 99% for the radiation emitted by the at least one first type LED. An example of such a glass is the longpass filter N-WG280 glass from Schott.

The material may be transparent for the radiation of the first type LED and the radiation of the second type LED. Optionally, the material is transparent PMMA or transparent glass allowing the radiation of the first type LED and the radiation of the second type LED to pass through.

In addition or alternatively, the optical diffuser may comprise a concave recess that is coaxial with the at least one first type LED. In addition or alternatively, the optical diffuser may comprise a concave recess that is coaxial with the at least one second type LED.

The respective one or more concave recesses may be formed on a first surface of the optical diffuser facing the at least one first type LED and the at least one second type LED, when the optical diffuser is arranged such that the radiation emitted by the first type LED and the radiation emitted by the second type LED passes through the optical diffuser. A respective concave recess of the optical diffuser formed on the aforementioned first surface of the optical diffuser and being coaxial with a LED (e.g. the first type LED or the second type LED) of the LED-module in combination with a second surface of the optical diffuser (opposing the first surface) acts as a diffuser lens. The second surface may be a flat surface or a plane surface. In other words, each concave recess of the optical diffuser is formed such that it acts as a diffuser lens for the LED with which it is coaxial.

Thus, the optical diffuser may comprise a diffuser lens coaxial with the at least one first LED. In addition or alternatively, the optical diffuser may comprise a diffuser lens coaxial with the at least one second LED.

The optical diffuser may be an optical diffuser sheet or optical diffuser plate. In this case, a concave recess may be formed on a first surface of the sheet or plate that is facing a respective LED (first type LED or second type LED) with which the concave recess is coaxial. The aforementioned concave recess acts then in combination with a second surface of the sheet or optical diffuser (opposing the first surface) as a diffuser lens for the respective LED.

The one or more concave recesses of the optical diffuser optically diffuse or scatter the respective radiation passing through it. Thus, the one or more concave recesses of the optical diffuser allow to prevent the at least one first type LED and/or the at least one second type LED from being visible as a single radiation point to a person looking at the LED-module.

Optionally, in case the LED-module comprises one first type LED, the optical diffuser comprises one concave recess (or diffuser lens) that is coaxial with the one first type LED. According to an embodiment, in case the LED-module comprise two or more first type LEDs, the optical diffuser comprises at least one concave recess (or at least one diffuser lens) that is coaxial with a respective first type LED of the first type LEDs. According to another embodiment, in case the LED-module comprise two or more first type LEDs, the optical diffuser comprises for each first type LED of the first type LEDs a concave recess (or diffuser lens) that is coaxial with the respective first type LED.

Optionally, in case the LED-module comprise one second type LED, the optical diffuser comprises one concave recess (or diffuser lens) that is coaxial with the one second type LED. According to an embodiment, in case the LED-module comprise two or more second type LEDs, the optical diffuser comprises at least one concave recess (or at least one diffuser lens) that is coaxial with a respective second type LED of the second type LEDs. According to another embodiment, in case the LED-module comprise two or more second type LEDs, the optical diffuser comprises for each second type LED of the second type LEDs a concave recess (or diffuser lens) that is coaxial with the respective second type LED.

In addition or alternatively, the optical diffuser may comprise a surface structure produced by sandblasting or acid etching. In other words, the optical diffuser may be translucent. For this, the optical diffuser may optionally be made of a transparent material, e.g. transparent PMMA or transparent glass, allowing the radiation emitted by the at least one first type LED and the radiation emitted by the at least one second type LED to pass through. The aforementioned transparent optical diffuser may be made translucent by sandblasting or acid etching. The surface structure may be according to the surface structure of frosted glass. That is, the optical diffuser may comprise a surface structure according to the surface structure of frosted glass. This may be produced by sandblasting or acid etching.

The optical diffuser being translucent, optionally due to sandblasting or acid etching the optical diffuser, allows improving the visual appearance of the LED-module significantly. Namely, due to the translucency of the optical diffuser (i.e. a person cannot see through the optical diffuser, as it is the case by frosted glass), the at least one first type LED and the at least one second type LED are not visible as single radiating points to a person, when they emit the respective radiation.

Alternatively or in addition, the LEDs of the first type and the second type can be, at least partly, arranged on opposite sides of the LED-module, preferably with the second type LEDs all on the same side. In case that the LED-module is used in a suspended luminaire it is thus possible to have a main direction in which white light is emitted by the luminaire, whereas into the opposite direction at least UV-B radiation is emitted. It has to be noted that it is not necessary that only second type LEDs are oriented to the opposite direction, but also LEDs of the first type can be arranged to emit light in the same direction. As a result, the LED-module or a luminaire comprising such LED-module generates direct and indirect white light for illuminating the room. However, the UV-B radiation emitted by the second type LEDs will be reflected by the ceiling and then propagate into the illuminated room only in a diffuse manner. Again, having only diffuse UV-B radiation reduces the risk of a local overexposure of the skin or eye of a human. Consequently, the risk of negatively affecting the health of a person is reduced.

According to a preferred embodiment, the second type LED, which is used in the inventive LED-module and lighting system, emits UV-B radiation with a peak wavelength between 300 nm and 350 nm, preferably between 303 nm and 313 nm. Further, the emitted UV-B radiation of the second type LED has a full width at half maximum below 25 nm, preferably below 20 nm. Selecting the LED of the second type according to the above-mentioned characteristics has the great advantage, that enhancement of the vitamin D production is possible without at the same time undesirably increasing health risk (mainly erythema) due to exposure of the human body to UV radiation. High doses of UV-B radiation can cause skin erythema and exposure over long time cancer, which, of course, must be avoided. For a second type LED having above-mentioned characteristics, the overlap of the vitamin D action spectrum and the radiation of the second type LED significantly exceeds the overlap between the second type LED radiation and a curve describing erythema risk (minimum erythema dose).

The first LED type may emit radiation having a peak wavelength between 445nm and 475nm, preferably between 455nm and 475nm.

The LED-module may comprise a light converting material including at least one component that can be excited by radiation emitted by the first type LED to emit light at least in the yellow, green or red color range. In case that the LED-module comprises a plurality of first type LEDs, the light converting material may be applied to all first type LEDs or only a portion of the first type LEDs depending on the desired characteristics of the generated white light.

In addition, the LED-module may comprise at least one further type LED. The further type LED may emit radiation in the green or red color range. The emission in the green or red color range may be achieved by using respective monochromatic LEDs or a suitable color converting material in combination with one or more blue LED. It is to be noted that even a plurality of further type LEDs can be combined to add green and red light components. Suitable color conversion materials for producing light in the green wavelength range, be a phosphor having a peak wavelength in the green wavelength range is used such as a garnet phosphor e.g. LuAG: Ce3+ or YAG: Ce3+(Y3Al5O12: Ce3+) or (Y, Gd)3 Al5O12:Ce3+)), an ortho- silicate e.g. (Ba2SiO4:Eu2+, (Ba,Sr)2SiO4:Eu2+) or β-SiAlON: Eu2+. For emitting light in the red wavelength range, suitable color conversion materials, which have a peak in the red wavelength range, are nitride ((Sr,Ca)AlSiN3: Eu2+ or CaAlSiN3: Eu2+) or KSF (K2SiF6: Mn4+). Instead of red phosphors red QDCC (quantum dot color conversion) can be used as well.

The optical diffuser may be arranged such that radiation emitted by the at least one first type LED, radiation emitted by the at least one second type LED and radiation emitted by the at least one further type LED passes through the optical diffuser. Thus, the optical diffuser may allow the radiation emitted by the at least one first type LED, the UV-B radiation and the radiation emitted by the at least one further type LED to pass or transmit through it, while optically diffusing or scattering these radiations. Therefore, the optical diffuser may allow the white light generated based on the radiation emitted by the at least one first type LED and the radiation emitted by the at least one further type LED to pass through. In other words, the optical diffuser may be implemented such that it does not absorb a significant amount of the radiation emitted by the at least one first type LED, the UV-B radiation and the radiation emitted by the at least one further type LED.

Optionally, only a part of the optical diffuser may allow the radiation emitted by the at least one first type LED, the UV-B radiation and the radiation emitted by the at least one further type LED to pass through it. That is, only a part of the optical diffuser may be made of a material allowing the radiation emitted by the at least one first type LED, the UV-B radiation and the radiation emitted by the at least one further type LED to pass through it. This improves costs because a material allowing the radiation emitted by the at least one first type LED, the UV-B radiation and the radiation emitted by the at least one further type LED may be expensive.

The optical diffuser may comprise a concave recess that is coaxial with the at least one further type LED. The optical diffuser may comprise a diffuser lens coaxial with the at least one further type LED. The above description with regard to the concave recess for the at least one first type LED and/or the at least one second type LED is correspondingly valid for the concave recess that is coaxial with the at least one further type LED.

The lighting system according to the invention comprises, in addition to the LED-module as described above, a control means configured to control the portion of UV-B radiation relative to the entire radiation emitted by the LED-module such that the UV-B radiation does not exceed an upper threshold. Limiting the UV-B radiation power to this upper threshold can be performed using sunlight as a reference together with the vitamin D action spectrum and the human eye's sensitivity for white light for defining the threshold. The minimal erythema dose (MED) may alternatively be used to estimate the upper threshold for UV-B radiation power. The MED can be estimated to be 20 mJ/cm². So if the upper threshold times the estimated maximum exposure time, for example eight working hours, equals the minimal erythema dose there is no relevant risk to suffer from erythema and the maximum exposure for enhancing production of vitamin D is achieved. It is especially preferred to adjust the portion of emitted UV-B radiation to match the upper threshold.

The threshold lies in the range from 180mW to 210mW, preferably in the range from 200mW to 210mW.

Further advantages and aspects of the invention may become clear from the following detailed description of the embodiments using the annexed drawings in which
- **Figure 1**: illustrates spectra of sunlight during summertime and wintertime,
- **Figure 2**: is a diagram showing the vitamin D action spectrum and an enlarged region of the sunlight spectrum together with the sensitivity of the human eye,
- **Figure 3**: illustrates resulting radiation power of UV-B radiation and light according to the spectra of figure 2,
- **Figure 4**: illustrates the comparison between the vitamin D action spectrum, minimal erythema dose, and an emission spectrum of the preferred UV-B LED,
- **Figure 5**: illustrates an embodiment of a lighting system according to the invention using a first inventive LED-module,
- **Figure 6**: illustrates an embodiment of a lighting system according to the invention using a second embodiment of the LED-module,
- **Figure 7A**: illustrates an embodiment of a lighting system according to the invention using a third embodiment of the LED-module,
- **Figure 7B**: illustrates an embodiment of a lighting system according to the invention using a fourth embodiment of the LED-module,
- **Figure 8A**: illustrates an embodiment of a lighting system according to the invention using a fifth embodiment of the LED-module,
- **Figure 8B**: illustrates an embodiment of a lighting system according to the invention using a sixth embodiment of the LED-module,
- **Figure 9A**: illustrates an embodiment of a lighting system according to the invention using a seventh embodiment of the LED-module, and
- **Figure 9B**: illustrates an embodiment of a lighting system according to the invention using an eighth embodiment of the LED-module.

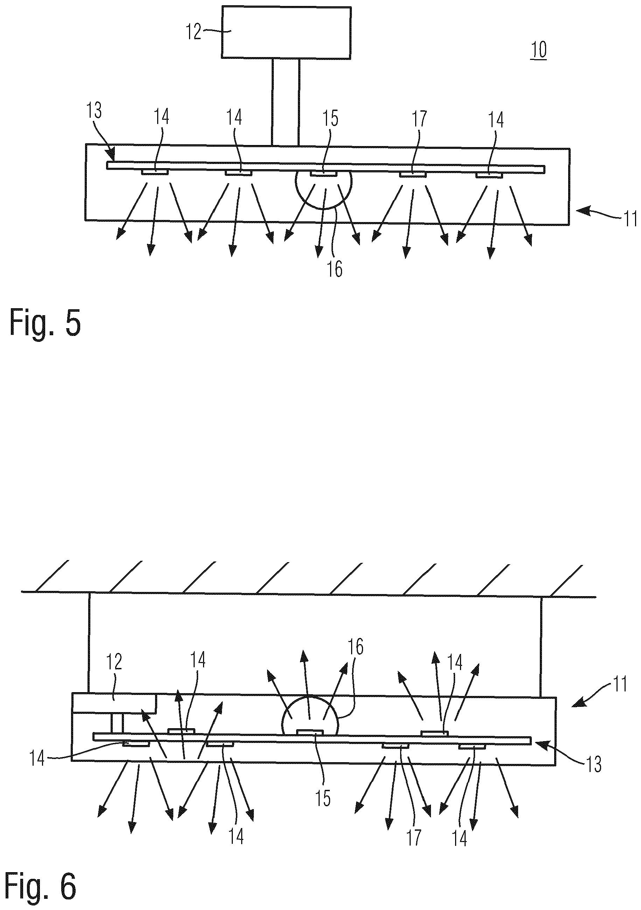

In the figures, corresponding elements have the same reference signs. The proportions and dimensions of the elements shown in the figures do not represent the LED-module and the luminaire to scale, but are merely chosen to describe the structure and function of the LED-module and the luminaire. The shape of the different elements of Figures 5, 6, 7A, 7B, 8A, 8B, 9A and 9B is only by way of example and may be differently.

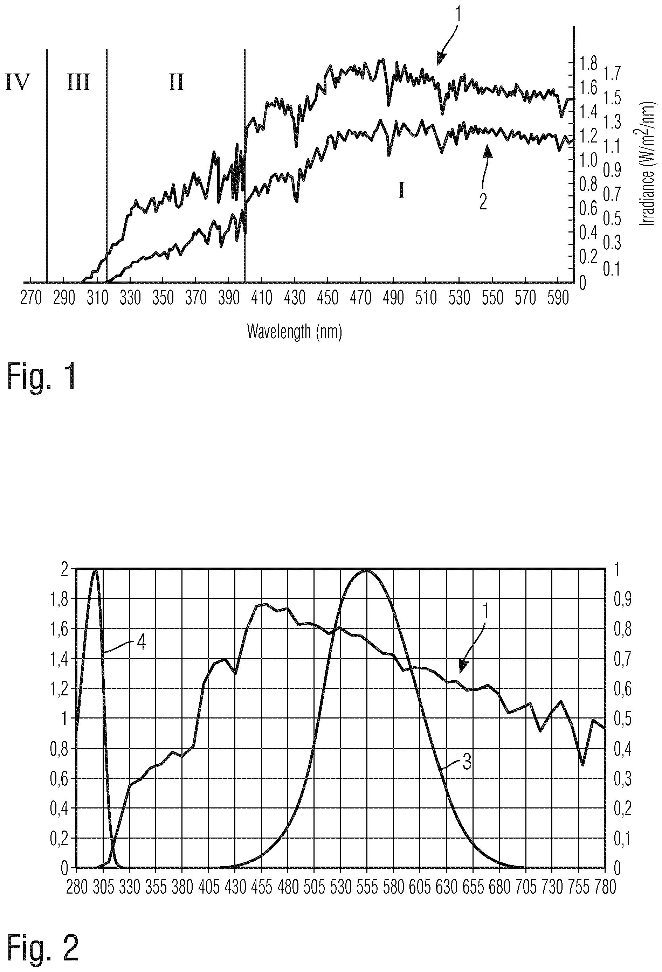

Figure 1 shows a first sunlight spectrum 1 which is typical for summertime and a second sunlight spectrum 2 during wintertime. It is to be noted that only a relevant portion of the spectra 1, 2 are shown in the diagram. The diagram denotes the irradiance over the wavelength in nanometers. The wavelength range is divided in a plurality of sub-ranges denoted with I, II, III and IV.

Region I extends from 400 nm to 600 nm and corresponds to a portion of the visible light, which means that radiation having such a wavelength is visible for the human eye. Nevertheless, the sensitivity of the human eye is not a constant value throughout the entire region I as it will be explained later with reference to figure 2.

Region II is the UV-A-region, which is not of particular relevance for the present invention and, thus, further elaboration on that region is omitted. Region III, adjacent to region II towards smaller wavelengths, is the area that is of specific interest with respect to the present invention, because this wavelength range is responsible for activating vitamin D production but also for causing erythema or even cancer. This region covers the wavelengths from 280 nm to 350 nm. Wavelengths below 280 nm belong to region IV, which is the so-called UV-C-region.

As can be seen directly in the diagram, the radiation power of summer sunlight 1 lies significantly above the radiation power of the winter sunlight 2. Further, it can be seen that during wintertime no significant radiation is present in region III, thus no UV-B radiation is available for the production of vitamin D in the skin of humans. Turning now to figure 2 it will become apparent that this seasonal effect of the radiation may result in a lack of vitamin D production during wintertime.

In addition to the section of the summertime spectrum 1 of sunlight, figure 2 also shows the sensitivity curve of the human eye, denoted with reference numeral 3 and the vitamin D action spectrum denoted with reference numeral 4. As it can be recognized, significant vitamin D production will be caused only by a small portion of the summer sunlight spectrum 1 towards small wavelengths. On the other hand, the sensitivity curve 3 results in perception of visible light also only in a small region of the entire sunlight spectrum.

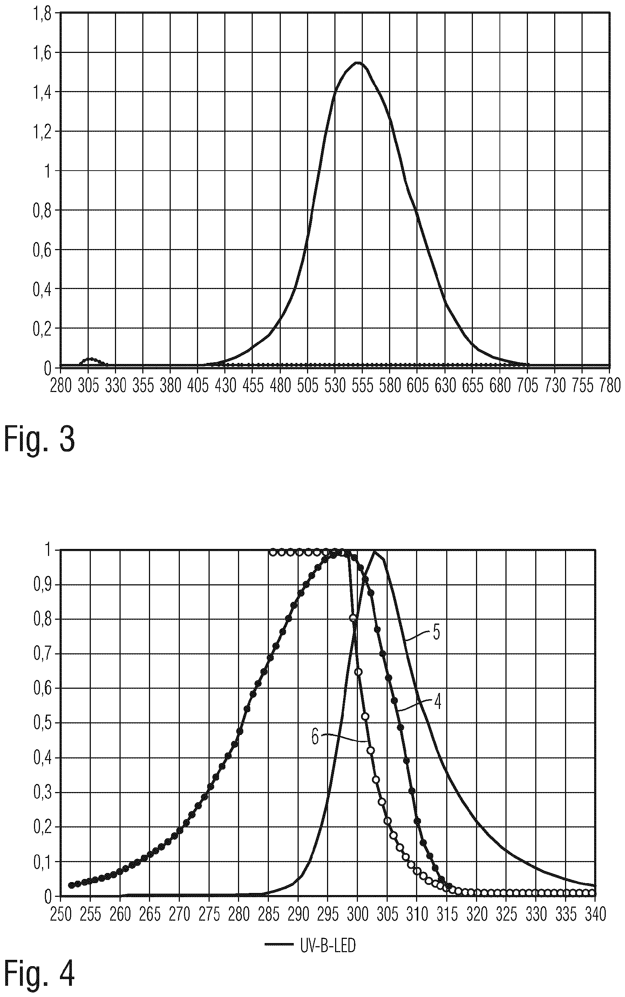

One approach for defining the UV-B radiation according to the invention is limiting the intensity of UV-B radiation and white light of natural sunlight perceivable by a human by a lighting system producing artificial light. Multiplying the sunlight spectrum 1 with the sensitivity curve 3 and multiplying the sunlight spectrum 1 with the vitamin D action spectrum 4 on the other hand results in the curves which are shown as dotted line for the vitamin D action spectrum and the photometric power curve shown as solid line in figure 3. These curves can now be used to estimate an upper threshold for the UV-B radiation. Based on the sunlight spectrum 1 as shown in figure 1, maximum illuminance on a summer day can be estimated to be 108,260 lux. The ratio of the UV-B power and the photometric power can be derived from the curves in figure 3 and is approximately 1,3:1000, which corresponds to 1,9mW/klm. With the estimated illuminance, this results in a UV-B power lying in the range from 180mW to 210mW, even more preferred in the range from 200mW to 210mW. It is most preferred that the threshold is 206mW corresponding to natural sunlight. Using natural sunlight as a reference for estimating the UV-B radiation power has the advantage, that automatically, because of the perceived brightness of white light, people will not directly look towards the light source. Thus, damage of the retina is avoided.

This estimated UV-B power may be used as upper threshold. However, it is preferred to set the UV-B power to equal the upper threshold.

In figure 4 the spectrum 5 of the preferred second type LED is shown together with the vitamin D action spectrum 4 and the minimal erythema dose 6. The diagram illustrates the advantage of the preferred second type LEDs having a peak wavelength between 300 and 315 nm, preferably between 303 and 313 nm, and a full width at half maximum below 25 mm, preferably below 20 nm. Using such a source for generating UV-B radiation automatically emphasizes the positive effect regarding vitamin D production in the skin over the risk of causing erythema. The overlap between the minimal erythema dose 6 and the spectrum of the second type LED is significantly less than the overlap between the second type LED spectrum 5 and the vitamin D action spectrum 4. Exploiting this shift between the curve of the minimal erythema dose 6 and the vitamin D action spectrum 4 by the specific selection of the UV-B radiation source allows to increase the UV-B radiation power such that a significant effect on vitamin D production is achieved without simultaneously increasing the risk for erythema.

Figure 5 shows a first example of a lighting system according to the present invention. The lighting system 10 comprises luminaire 11, which is controlled by control means 12. The control means 12 is electrically connected to an LED-module 13 comprising a plurality of first type LEDs 14 and at least one second type LEDs 15, and provides a controlled current for operating the LEDs 14 and 15. The first type LEDs 14 emit radiation, which is suitable to generate white light based thereon. It is to be noted, that not only one single type of LEDs 14 may be used in order to generate the desired white light, but also combination of different LED types which suitable to generate white light. For example, additive color mixing may require a plurality of different LED. The combination of different types of LEDs defines the characteristics, in particular the color temperature of the resulting white light.

As an example, one further type LED 17 is shown in the drawing. The further type LED may emit radiation in the green or red color range. The emission in the green or red color range may be achieved by using respective monochromatic LEDs or a suitable color converting material in combination with one or more blue LED. It is to be noted that even a plurality of further type LEDs can be combined to add green and red light components. Suitable color conversion materials for producing light in the green wavelength range, be a phosphor having a peak wavelength in the green wavelength range is used such as a garnet phosphor e.g. LuAG: Ce3+ or YAG: Ce3+(Y3Al5O12: Ce3+) or (Y, Gd)3 Al5O12:Ce3+)), an ortho- silicate e.g. (Ba2SiO4:Eu2+, (Ba,Sr)2SiO4:Eu2+) or β-SiAlON: Eu2+. For emitting light in the red wavelength range, suitable color conversion materials, which have a peak in the red wavelength range, are nitride ((Sr,Ca)AlSiN3: Eu2+ or CaAlSiN3: Eu2+) or KSF (K2SiF6: Mn4+). Instead of red phosphors red QDCC (quantum dot color conversion) can be used as well

In contrast to the radiation emitted by the first type LEDs 14 and, if any, additionally the radiation emitted by the further type LEDs, the radiation emitted by the second type LEDs 15 does not contribute to the emission of white light. Thus, the radiation produced by the second type LEDs 15 is not intended for illuminating a room but is dedicated UV-B radiation for being emitted by the LED-module 13 in order to enhance vitamin D production in the skin. The radiation power emitted by the second type LED 15 (or a plurality of second type LEDs 15) is controlled by the control means 12, which is preferably capable of individually setting the current for the first type LEDs 14 and second type LEDs 15. Alternatively, in case that the current may be controlled only for the first type LEDs 14 and the second type LEDs 15, the portion of UV-B radiation power relative to radiation power of the generated white light can be adjusted by determining the number of first type LEDs 14 and second type LEDs 15.

In order to avoid an overdose caused by the UV-B radiation emitted by the LEDs 15, a diffuser 16 may be applied. The diffuser 16 preferably only diffuses the emitted radiation of the second type LEDs 15. It is preferred that the diffuser 16 is attached to the LED-module 13 in such a way that it covers the second type LED 15. In an alternative embodiment it is also possible to commonly diffuse the radiation emitted by the first type LEDs 14 and the second type LEDs 15. In that case, it would also be possible to use a diffuser which is part of the luminaire and does not need to be directly applied on the LED-module 13. The material must then be chosen appropriately such that the material does not show a significant absorption of UV radiation.

The first type LEDs 14 and the second type LEDs 15 are arranged on the same PCB (printed circuit board). In order to improve efficiency, it is desired that the PCB has a high reflectivity, which can be achieved by using a solder mask with good reflectivity in the range of 250 nm and 800 nm. For example, a PTFE plastic coating could be used or an additional sheet made of aluminum or PTFE plastic. The PTFE plastics reflectivity is above 95%. Aluminum's reflectivity is above 70% in the complete wavelength range (250 nm to 800 nm). Additionally, the sidewalls of the entire luminaire 11, or at least parts thereof, may also be coated with such a material or a reflector could be applied.

A cover of the luminaire, which may also be used as a diffuser, needs to have a high transmittance in the complete wavelength range (250 nm to 800 nm). To avoid absorption of radiation having a wavelength below 400 nm, which is typical for generally used cover materials like PMMA or PC (polycarbonate), preferred materials could be quartz glass or UV grade silicone. For quartz glass, the transmission is nearly 100% in the complete range and for UV grade silicone the transmission rate lies around 95% for the complete wavelength range.

To improve the appearance of the luminaire 11, it is desirable that the cover is not entirely transparent. Thus, in order to avoid that the interior of the luminaire 11 is visible from the outside, particles or additives (light diffusion agent) should be added to the cover material.

The radiation power of the first type LEDs 14 and the one or more second type LED 15 can be controlled using the control means 12, as mentioned above. Thus, the LED-module 13 may be dimmable. In order not to weaken the effect of the emitted UV-B radiation, it is preferred that the UV-B power is set to a constant value, preferably the upper threshold even in case that the luminaire 11 is dimmed.

A second embodiment of the lighting system 10 according to the present invention is illustrated in figure 6. It is to be noted that same reference numerals denote same or corresponding components. The major difference between the embodiment shown in figure 5 and explained above and second embodiment of figure 6 is that the luminaire 11 according to the second embodiment comprises an LED-module 13 having LEDs 14 and 15 with opposite radiation directions. The main direction for emitting white light generated based on the radiation of the first type LEDs 14 is intended to produce direct light for illuminating the room. In addition, (a preferably smaller number of) LEDs 14 and in particular the second type LED 15 are arranged on the LED-module 13 such that their radiation direction goes to the opposite direction. Thus, when the LEDs 14 of the first type are used to produce direct light for illuminating a room, the other LEDs 14 and particularly the second type LEDs 15 produce indirect light. While such indirect light produced by the first type LEDs 14 is only relevant for appearance and design, emitting indirect UV-B radiation has the great advantage that the UV-B radiation is reflected on the optically rough ceiling and propagation into the room is diffuse.

According to a preferred embodiment, the first type LEDs 14 and, where applicable, the further type LEDs 17), are all arranged on a first side of the PCB of the LED-module 13. The second type LEDs 15 are only arranged on the opposite surface. Thus, commonly known and well established materials forming the cover and/or diffuser can be attached to the luminaire 11, thereby achieving a desirable appearance of the luminaire 11. On the other hand, the other side of the luminaire, through which the UV radiation is emitted, can be optimized for UV radiation transparency. The diffuser 16 and maybe further optical elements are designed such that radiation characteristics for the UV radiation is similar to the radiation pattern of the visible light source (first type LEDs 14 and further type LEDs 17) in order to enable an appropriate lighting design. The further optical elements (reflectors and/ or lenses) are designed to improve the efficiency, because they can be designed to direct the emitted UV radiation of the second type LEDs 15 such that the reflected radiation from the ceiling is not absorbed by the luminaire 11.

Such indirect UV-B radiation can advantageously be combined with the diffuser 16 in order to even increase the effect of diffuse radiation.

The embodiment shown in figure 5 shows the control means 12 arranged externally to the luminaire 11 in order to establish the lighting system 10. However, as it is shown in figure 6, the control means 12 may also be integrated in the luminaire 11. In both embodiments, the luminaire 11 may be a suspended luminaire as explicitly illustrated in figure 6.

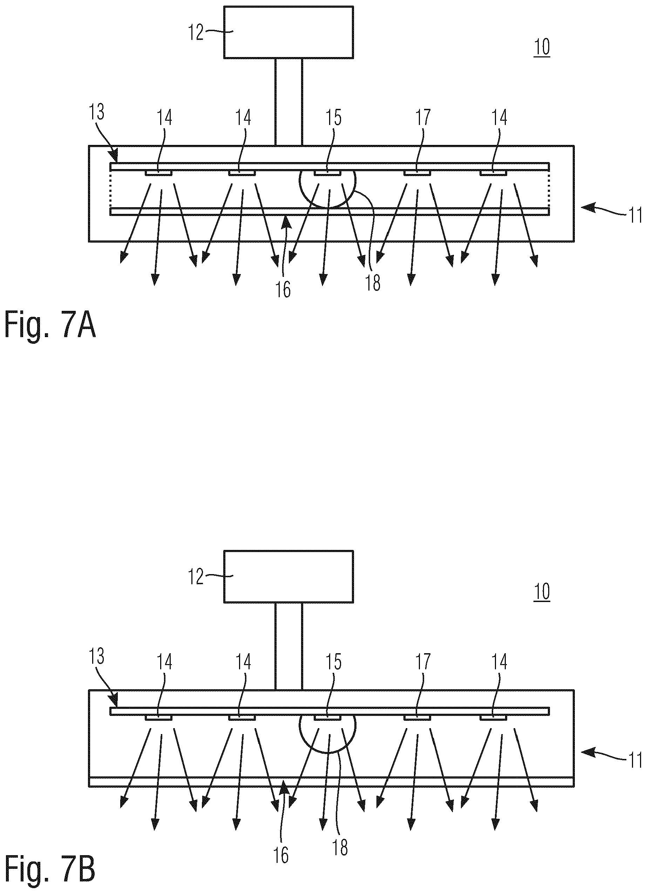

Figure 7A illustrates a third embodiment of the lighting system 10 according to the present invention using a third embodiment of the LED-module 13. The lighting system 10 of figure 7A corresponds to the lighting system 10 of figure 5, wherein the implementation of the optical diffuser 16 of the LED-module 13 is different. Thus, the description of the lighting system 10 of figure 5 is correspondingly valid for the lighting system 10 of figure 7A and in the following mainly the differences are described.

As shown in figure 7A, the optical diffuser 16 is arranged such that radiation emitted by the first type LEDs 14, the radiation emitted by the optional one further type LED 17 and radiation emitted by the at least one second type LED 15 passes through the optical diffuser 16. Optionally, the optical diffuser 16 is attached to the PCB of the LED-module 13, on which the first type LEDs 14, the at least one second type LED 15 and the optional further type LED 17 are arranged (indicated by the dashed lines).

Optionally, the LED-module 13 may comprise one or more optional further optical elements 18 for the at least one second type LED 15, as schematically indicated in figure 7A. The one or more optional further optical elements 18 are designed such that radiation characteristics for the UV-B radiation is similar to the radiation pattern of the visible light source (first type LEDs 14 and optional further type LED 17) in order to enable an appropriate lighting design. The optional one or more further optical elements may be at least one reflector and/or at least one lens.

The optical diffuser 16 allows the radiation emitted by the first type LEDs 14, the radiation emitted by the optional further type LED 17 and the UV-B radiation emitted by the at least one second type LED 15 to pass or transmit through it, while optically diffusing or scattering these radiations. Therefore, the optical diffuser allows the white light, generated based on the radiation emitted by the first type LEDs 15 and the radiation emitted by the optional further type LED 17, to pass through.

The optical diffuser 16 may be made of a material having a transmittance in a wavelength range from 250 nm to 800 nm or a material having a transmittance in a wavelength from 300 nm to 600 nm. The material may be PMMA or glass. According to an embodiment, the optical diffuser 16 may be made of PMMA having a transmittance in a wavelength range from 250 nm to 800 nm or in a wavelength range from 300 nm to 600 nm. According to another embodiment, the optical diffuser 16 may be made of glass having a transmittance in a wavelength range from 250 nm to 800 nm or in a wavelength range from 300 nm to 600 nm. The glass may have an internal transmittance of at least 90% for the UV-B radiation and an internal transmittance of at least 99% for the radiation emitted by the first type LEDs 15 and the optional further type LED 17.

Optionally, the optical diffuser 16 may comprise a surface structure produced by sandblasting or acid etching. In other words, the optical diffuser 16 may be translucent. For this, the optical diffuser 16 may optionally be made of a transparent material, e.g. transparent PMMA or transparent glass, allowing the radiation emitted by the first type LEDs 15, the UV-B radiation and the radiation emitted by the optional further type LED 17 to pass through. The transparent optical diffuser 16 may be made translucent by sandblasting or acid etching. The surface structure may be according to the surface structure of frosted glass. That is, the optical diffuser 16 may comprise a surface structure according to the surface structure of frosted glass. This may be produced by sandblasting or acid etching.

For further information on the optical diffuser 16 reference is made to the above description of an optical diffuser used in an inventive LED-module. For further information on the lighting system 10 of figure 7A reference is made to the description of the lighting system 10 of figure 5.

Figure 7B illustrates a fourth embodiment of the lighting system 10 according to the present invention using a fourth embodiment of the LED-module 13. The lighting system 10 of figure 7B corresponds to the lighting system 10 of figure 7A, wherein the implementation of the optical diffuser 16 is different. Thus, the description of the lighting system 10 of figures 5 and 7A is correspondingly valid for the lighting system 10 of figure 7B and in the following mainly the differences are described.

As shown in figure 7B, the optical diffuser 16 may also be an external component with regard to the LED-module 13, i.e. the optical diffuser 16 may not be comprised by the LED-module 13. In this case, the optical diffuser 16 is a part of the luminaire 11. That is, the luminaire 11 comprises the LED-module 13 and the optical diffuser 16, wherein the optical diffuser 16 is arranged such that radiation emitted by the first type LEDs 14, the radiation emitted by the optional one further type LED 17 and UV-B radiation emitted by the at least one second type LED 15 passes through the optical diffuser 16.

For further information on the lighting system 10, in particular the optical diffuser 16, of figure 7B reference is made to the description of the lighting system 10, in particular the optical diffuser 16, of figure 7A.

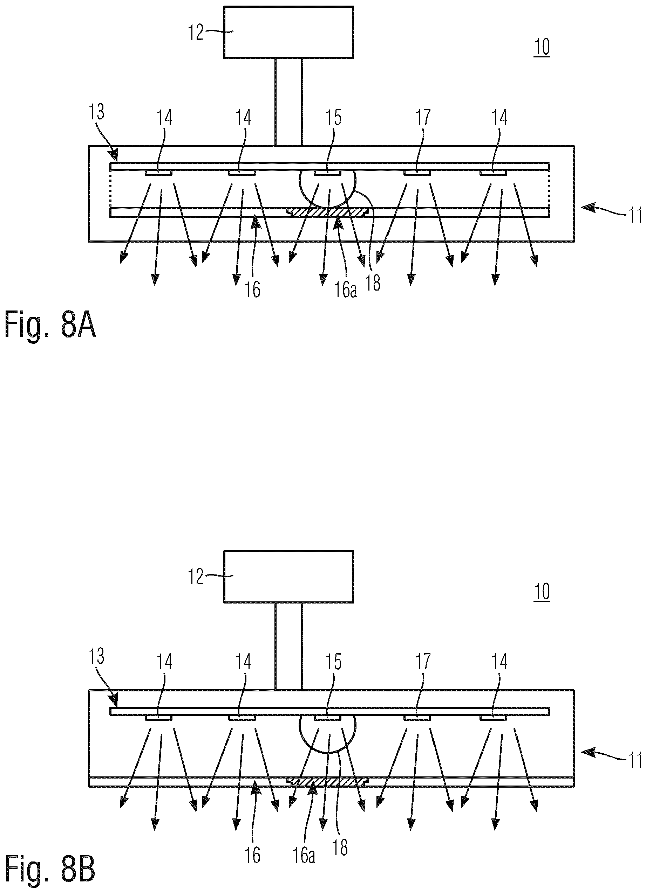

Figure 8A illustrates a fifth embodiment of the lighting system 10 according to the present invention using a fifth embodiment of the LED-module 13. The lighting system 10 of figure 8A corresponds to the lighting system 10 of figure 7A, wherein the implementation of the optical diffuser 16 is different. Thus, the description of the lighting system 10 of figures 5 and 7A is correspondingly valid for the lighting system 10 of figure 8A and in the following mainly the differences are described.

As indicated in figure 8A, only the part 16a of the optical diffuser 16 for the UV-B radiation emitted by the at least one second type LED 15 may be made of the material having a transmittance in a wavelength range from 250 nm to 800 nm or the material having a transmittance in a wavelength from 300 nm to 600 nm. The aforementioned part 16a of the optical diffuser 16 is hatched in figure 8A. The shape of the aforementioned part 16a of the optical diffuser 16 shown in figure 8A is only by way of example and, thus, may be differently implemented. The material may be PMMA or glass. According to an embodiment, the part 16a of the optical diffuser 16 may be made of PMMA having a transmittance in a wavelength range from 250 nm to 800 nm or in a wavelength range from 300 nm to 600 nm. According to another embodiment, the part 16a of the optical diffuser 16 may be made of glass having a transmittance in a wavelength range from 250 nm to 800 nm or in a wavelength from 300 nm to 600 nm. The glass may have an internal transmittance of at least 90% for the UV-B radiation and an internal transmittance of at least 99% for the radiation emitted by the first type LEDs 15 and the optional further type LED 17.

The rest of the optical diffuser 16 may be made of a material allowing the radiation emitted by the first type LEDs 14 and the radiation emitted by the optional further type LED 17 to pass through it. This rest of the optical diffuser 16 does not need to allow the UV-B radiation to pass through. As a result, the costs for the optical diffuser 16 of figure 8A may be lower than the costs for the optical diffuser 16 of 7A, because the amount of the material having a transmittance in the wavelength range from 250 nm to 800 nm or from 300 nm to 600 nm needed for implementing the part 16a of the optical diffuser 16 of figure 8A is smaller than the amount of that material needed for implementing the whole optical diffuser 16 of figure 7A. Such a material may be expensive. The implementation of the optical diffuser 16 of figure 8A may be done by segmenting the optical diffuser 16 in order to create the part 16a, through which the UV-B radiation may be emitted.

The sandblasting or acid etching, described above with regard to figure 7A, may optionally be performed for the whole optical diffuser 16. Thus, the optical diffuser 16 may be translucent. The optical diffuser 16 may have a surface structure according to the surface structure of frosted glass.

For further information on the lighting system 10, in particular the optical diffuser 16, of figure 8A reference is made to the description of the lighting system 10, in particular the optical diffuser 16, of figure 7A.

Figure 8B illustrates a sixth embodiment of the lighting system 10 according to the present invention using a sixth embodiment of the LED-module 13. The lighting system 10 of figure 8B corresponds to the lighting system 10 of figure 8A, wherein the implementation of the optical diffuser 16 is different. Thus, the description of the lighting system 10 of figure 8A is correspondingly valid for the lighting system 10 of figure 8B and in the following mainly the differences are described.

As shown in figure 8B, the optical diffuser 16 may also be an external component with regard to the LED-module 13, i.e. the optical diffuser 16 may not be comprise by the LED-module 13. In this case, the optical diffuser 16 is a part of the luminaire 11. That is, the luminaire 11 comprises the LED-module 13 and the optical diffuser 16, wherein the optical diffuser 16 is arranged such that radiation emitted by the first type LEDs 14, the radiation emitted by the optional further type LED 17 and radiation emitted by the at least one second type LED 15 passes through the optical diffuser 16.

For further information on the lighting system 10, in particular the optical diffuser 16, of figure 8B reference is made to the description of the lighting system 10, in particular the optical diffuser 16, of figure 8A.

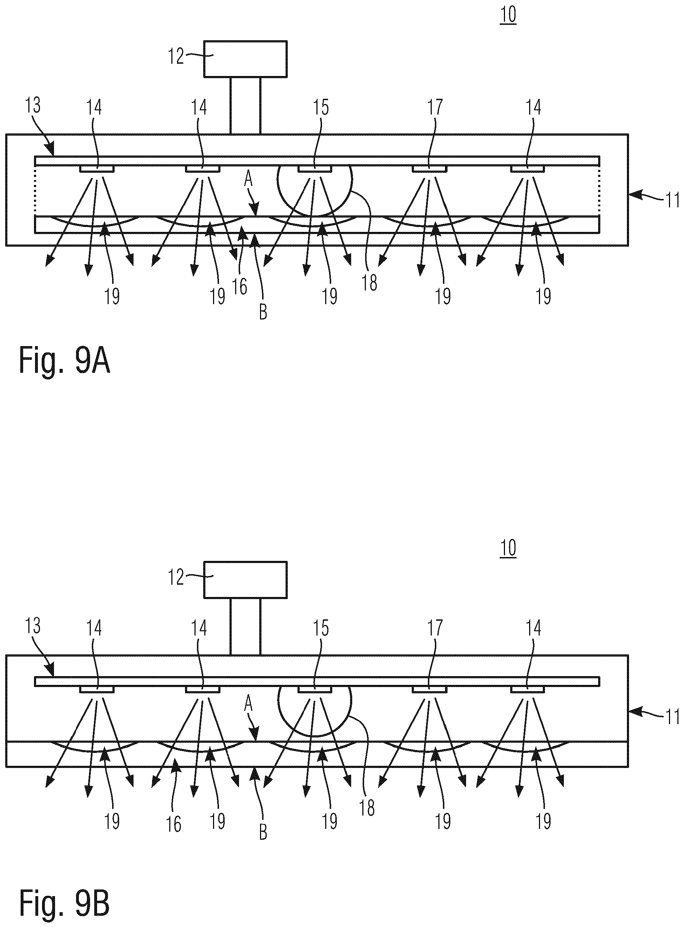

Figure 9A illustrates a seventh embodiment of the lighting system 10 according to the present invention using a seventh embodiment of the LED-module 13. The lighting system 10 of figure 9A corresponds to the lighting system 10 of figure 7A, wherein the implementation of the optical diffuser 16 is different. Thus, the description of the lighting system 10 of figures 5 and 7A is correspondingly valid for the lighting system 10 of figure 9A and in the following mainly the differences are described.

As indicated in figure 9A, the optical diffuser 16 may comprise a concave recess 19 (or diffuser lens) for a first type LED 14, a second type LED 15 and/or an optional further type LED 17, wherein the concave recess 19 is coaxial with the respective LED. According to an example, which is shown in figure 9A, the optical diffuser 16 comprises for each LED of the first type LEDs 14, the at least one second type LED 15 and the optional further type LED 17 a concave recess 19 (or diffuser lens) that is coaxial with the respective LED. The concave recesses 19 are formed on a first surface A of the optical diffuser 16, the first surface facing the first type LEDs 14 and the at least one second type LED 15. These concave recesses 19 in combination with the flat or plane second surface B of the optical diffuser 16, the second surface B opposing the first surface A, act as diffuser lenses. Thus, each concave recess 19 coaxial with a respective LED (e.g. first type LED 14, second type LED 15 or optional further type LED 17) diffuses or scatters the radiation emitted by the respective LED.

The optional implementation of the optical diffuser 16 of the lighting system 10 of figure 8A may be combined with the implementation of concave recesses 19 (or diffuser lenses) as exemplarily shown in figure 9A and described above.

For further information on the lighting system 10, in particular the optical diffuser 16, of figure 9A reference is made to the description of the lighting system 10, in particular the optical diffuser 16, of figure 7A.

Figure 9B illustrates an eighth embodiment of the lighting system 10 according to the present invention using an eighth embodiment of the LED-module 13. The lighting system 10 of figure 9B corresponds to the lighting system 10 of figure 9A, wherein the implementation of the optical diffuser 16 is different. Thus, the description of the lighting system 10 of figure 9A is correspondingly valid for the lighting system 10 of figure 9B and in the following mainly the differences are described.

As shown in figure 9B, the optical diffuser 16 may also be an external component with regard to the LED-module 13, i.e. the optical diffuser 16 may not be comprised by the LED-module 13. In this case, the optical diffuser 16 is a part of the luminaire 11. That is, the luminaire 11 comprises the LED-module 13 and the optical diffuser 16, wherein the optical diffuser 16 is arranged such that radiation emitted by the first type LEDs 14, the radiation emitted by the optional further type LED 17 and radiation emitted by the at least one second type LED 15 passes through the optical diffuser 16.

For further information on the lighting system 10, in particular the optical diffuser 16, of figure 9B reference is made to the description of the lighting system 10, in particular the optical diffuser 16, of figure 9A.

In the embodiments of figures 7A, 7B, 8A, 8B, 9A and 9B, at least one second type LED 15 may be arranged on a side (second side) of the PCB of the LED-module 13 that is opposite to a side (first side) of the PCB, on which first type LEDs 14, at least one second type LED 15 and the optional further type LED 17 are arranged. For this case, the description of the lighting system 10 of figure 6 may be correspondingly valid for the lighting system 10 of figures 7A, 7B, 8A, 8B, 9A and 9B.

The embodiments shown in figures 7A, 7B, 8A, 8B, 9A and 9B show the control means 12 arranged externally to the luminaire 11 in order to establish the lighting system 10. However, as it is shown in figure 6, the control means 12 may also be integrated in the luminaire 11. In the embodiments shown in figures 7A, 7B, 8A, 8B, 9A and 9B, the luminaire 11 may be a suspended luminaire as explicitly illustrated in figure 6.

## Claims

1. Lighting system (10) comprising at least one LED-module (13) and control means (12), the LED-module (13) comprising at least one first type LED (14) emitting radiation suitable for generating white light, and at least one second type LED (15) emitting UV-B radiation, wherein
the LED-module (13) is configured to emit white light and UV-B radiation,
the control means (12) is configured to control the portion of UV-B radiation power relative to the entire radiation power such that the UV-B radiation does not exceed an upper threshold, **characterised in that**
the upper threshold is in the range from 18omW to 210mW, preferably in the range from 200mW to 210mW, and most preferred 206mW.

2. Lighting system (10) according to claim 1, wherein the LED-module (13) comprises an optical diffuser (16) arranged such that radiation emitted by the at least one second type LED (15) passes through the optical diffuser (16).

3. Lighting system (10) according to claim 2, wherein the optical diffuser (16) is arranged such that radiation emitted by the at least one first type LED (14) and radiation emitted by the at least one second type LED (15) passes through the optical diffuser (16).

4. Lighting system (10) according to claim 2 or 3, wherein at least a part (16a) of the optical diffuser (16) for the radiation emitted by the at least one second type LED (15), optional the optical diffuser (16), is made of a material having a transmittance in a wavelength range from 250 nm to 800 nm, optionally from 300 nm to 600 nm.

5. Lighting system (10) according to claim 4, wherein the material is PMMA or glass.

6. Lighting system (10) according to any one of claims 2 to 5, wherein the optical diffuser (16) comprises a concave recess (19) that is coaxial with the at least one first type LED (14) and/or a concave recess (19) that is coaxial with the at least one second type LED (15).

7. Lighting system (10) according to any one of claims 2 to 6, wherein the optical diffuser comprises a surface structure produced by sandblasting or acid etching.

8. Lighting system (10) according to any one of claims 1 to 7, wherein at least one first type LED (14) and at least one second type LED (15) are arranged to emit their respective radiation into opposite directions of the LED-module (13).

9. Lighting system (10) according to any one of claims 1 to 8, wherein the at least one second type LED (15) emits radiation having a peak wavelength between 300 and 315 nm, preferably between 303 and 313 nm, and a full width at half maximum below 25 mm, preferably below 20 nm.

10. Lighting system (10) according to any one of claims 1 to 9, wherein the at least one first type LED (14) emits radiation with a peak wavelength between 445 and 475 nm, preferably between 455 and 475.

11. Lighting system (10) according to any one of claims 1 to 10, wherein the LED-module comprises a color converting material including at least one component that can be excited by radiation emitted by the first type LED (14) to emit light at least in the yellow, green or red color range.

12. Lighting system (10) according to any one of claims 1 to 11, wherein the LED-module comprises at least one further type LED emitting radiation in the green or red color range.

13. Lighting system (10) according to any one of claims 1 to 12, wherein the lighting system (10) is a luminaire (11).

## Patentansprüche

1. Beleuchtungssystem (10), umfassend mindestens ein LED-Modul (13) und Steuermittel (12), das LED-Modul (13) umfassend mindestens eine LED (14) des ersten Typs, die Strahlung emittiert, die zum Generieren von weißem Licht geeignet ist, und mindestens eine LED (15) des zweiten Typs, die UV-B-Strahlung emittiert, wobei
das LED-Modul (13) konfiguriert ist, um weißes Licht und UV-B-Strahlung zu emittieren, die Steuereinrichtung (12) konfiguriert ist, um den Anteil der UV-B-Strahlungsleistung relativ zu der Gesamtstrahlungsleistung derart zu steuern, dass die UV-B-Strahlung einen oberen Schwellenwert nicht überschreitet,
**dadurch gekennzeichnet, dass** der obere Schwellenwert in dem Bereich von 180 mW bis 210 mW, vorzugsweise in dem Bereich von 200 mW bis 210 mW und am meisten bevorzugt bei 206 mW liegt.

2. Beleuchtungssystem (10) nach Anspruch 1, wobei das LED-Modul (13) einen optischen Diffusor (16) umfasst, der derart angeordnet ist, dass Strahlung, die durch die mindestens eine LED (15) des zweiten Typs emittiert wird, durch den optischen Diffusor (16) hindurchtritt.

3. Beleuchtungssystem (10) nach Anspruch 2, wobei der optische Diffusor (16) derart angeordnet ist, dass Strahlung, die durch die mindestens eine LED des ersten Typs (14) emittiert wird, und Strahlung, die durch die mindestens eine LED des zweiten Typs (15) emittiert wird, durch den optischen Diffusor (16) hindurchtreten.

4. Beleuchtungssystem (10) nach Anspruch 2 oder 3, wobei mindestens ein Teil (16a) des optischen Diffusors (16) für die Strahlung, die durch die mindestens eine LED (15) zweiten Typs emittiert wird, optional der optische Diffusor (16), aus einem Material gefertigt ist, das eine Durchlässigkeit in einem Wellenlängenbereich von 250 nm bis 800 nm, optional von 300 nm bis 600 nm aufweist.

5. Beleuchtungssystem (10) nach Anspruch 4, wobei das Material PMMA oder Glas ist.

6. Beleuchtungssystem (10) nach einem der Ansprüche 2 bis 5, wobei der optische Diffusor (16) eine konkave Aussparung (19), die koaxial zu der mindestens einen LED des ersten Typs (14) ist, und/oder eine konkave Aussparung (19), die koaxial zu der mindestens einen LED des zweiten Typs (15) ist, umfasst.

7. Beleuchtungssystem (10) nach einem der Ansprüche 2 bis 6, wobei der optische Diffusor eine Oberflächenstruktur, die durch Sandstrahlen oder Säureätzen erzeugt wird, umfasst.

8. Beleuchtungssystem (10) nach einem der Ansprüche 1 bis 7, wobei mindestens eine LED des ersten Typs (14) und mindestens eine LED des zweiten Typs (15) angeordnet sind, um ihre jeweilige Strahlung in entgegengesetzte Richtungen des LED-Moduls (13) zu emittieren.

9. Beleuchtungssystem (10) nach einem der Ansprüche 1 bis 8, wobei die mindestens eine LED (15) des zweiten Typs Strahlung, die eine Spitzenwellenlänge zwischen 300 und 315 nm, vorzugsweise zwischen 303 und 313 nm, und eine Halbwertsbreite von unter 25 mm, vorzugsweise unter 20 nm aufweist, emittiert.

10. Beleuchtungssystem (10) nach einem der Ansprüche 1 bis 9, wobei die mindestens eine LED (14) des ersten Typs Strahlung, die eine Spitzenwellenlänge zwischen 445 und 475 nm, vorzugsweise zwischen 455 und 475 nm aufweist, eimittiert.

11. Beleuchtungssystem (10) nach einem der Ansprüche 1 bis 10, wobei das LED-Modul ein farbkonvertierendes Material umfasst, einschließlich mindestens einer Komponente, die durch Strahlung, die durch die LED (14) des ersten Typs emittiert wird, angeregt werden kann, um Licht mindestens in dem gelben, grünen oder roten Farbbereich zu emittieren.

12. Beleuchtungssystem (10) nach einem der Ansprüche 1 bis 11, wobei das LED-Modul mindestens einen weiteren LED-Typ umfasst, der Strahlung in dem grünen oder roten Farbbereich emittiert.

13. Beleuchtungssystem (10) nach einem der Ansprüche 1 bis 12, wobei das Beleuchtungssystem (10) eine Leuchte (11) ist.

## Revendications

1. Système d'éclairage (10) comprenant au moins un module de DEL (13) et un moyen de commande (12), le module de DEL (13) comprenant au moins une DEL de premier type (14) émettant un rayonnement adapté à la production de lumière blanche, et au moins une DEL de second type (15) émettant un rayonnement UV-B, dans lequel
le module de DEL (13) est configuré pour émettre une lumière blanche et un rayonnement UV-B, le moyen de commande (12) est configuré pour commander la partie de puissance de rayonnement UV-B par rapport à la puissance de l'ensemble des rayonnements de sorte que le rayonnement UV-B ne dépasse pas un seuil supérieur, **caractérisé en ce que** le seuil supérieur est compris entre 180 mW et 21o mW, de préférence entre 200 mW et 21o mW, et le plus préférablement égal à 206 mW.

2. Système d'éclairage (10) selon la revendication 1, dans lequel le module de DEL (13) comprend un diffuseur optique (16) agencé de manière à ce que le rayonnement émis par l'au moins une DEL de second type (15) passe à travers le diffuseur optique (16).

3. Système d'éclairage (10) selon la revendication 2, dans lequel le diffuseur optique (16) est agencé de manière à ce que le rayonnement émis par l'au moins une DEL de premier type (14) et le rayonnement émis par l'au moins une DEL de second type (15) passent à travers le diffuseur optique (16).

4. Système d'éclairage (10) selon la revendication 2 ou 3, dans lequel au moins une partie (16a) du diffuseur optique (16) pour le rayonnement émis par l'au moins une DEL de second type (15), éventuellement le diffuseur optique (16), est faite d'un matériau ayant une transmittance dans une plage de longueur d'onde de 250 nm à 800 nm, éventuellement de 300 nm à 600 nm.

5. Système d'éclairage (10) selon la revendication 4, dans lequel le matériau est du PMMA ou du verre.

6. Système d'éclairage (10) selon l'une quelconque des revendications 2 à 5, dans lequel le diffuseur optique (16) comprend un évidement concave (19) coaxial avec l'au moins une DEL de premier type (14) et/ou un évidement concave (19) coaxial avec l'au moins une DEL de second type (15).

7. Système d'éclairage (10) selon l'une quelconque des revendications 2 à 6, dans lequel le diffuseur optique comprend une structure de surface produite par sablage ou gravure à l'acide.

8. Système d'éclairage (10) selon l'une quelconque des revendications 1 à 7, dans lequel au moins une DEL de premier type (14) et au moins une DEL de second type (15) sont agencées pour émettre leur rayonnement respectif dans des directions opposées du module de DEL (13).

9. Système d'éclairage (10) selon l'une quelconque des revendications 1 à 8, dans lequel l'au moins une DEL de second type (15) émet un rayonnement dont la longueur d'onde maximale est comprise entre 300 et 315 nm, de préférence entre 303 et 313 nm, et dont la largeur maximale à mi-hauteur est inférieure à 25 mm, de préférence inférieure à 20 nm.

10. Système d'éclairage (10) selon l'une quelconque des revendications 1 à 9, dans lequel l'au moins une DEL de premier type (14) émet un rayonnement dont la longueur d'onde maximale est comprise entre 445 et 475 nm, de préférence entre 455 et 475.

11. Système d'éclairage (10) selon l'une quelconque des revendications 1 à 10, dans lequel le module de DEL comprend un matériau de conversion de couleur comportant au moins un composant qui peut être excité par le rayonnement émis par la DEL de premier type (14) pour émettre une lumière au moins dans la gamme de couleurs jaune, vert ou rouge.

12. Système d'éclairage (10) selon l'une quelconque des revendications 1 à 11, dans lequel le module de DEL comprend au moins un autre type de DEL émettant un rayonnement dans la gamme de couleurs verte ou rouge.

13. Système d'éclairage (10) selon l'une quelconque des revendications 1 à 12, dans lequel le système d'éclairage (10) est un luminaire (11).
